(19) Europäisches Patentamt
European Patent Office
Office européen des brevets

(11) Veröffentlichungsnummer: **0 071 018**
**B1**

# (12) EUROPÄISCHE PATENTSCHRIFT

(45) Veröffentlichungstag der Patentschrift: **08.01.86**

(51) Int. Cl.⁴: **C 07 D 239/55**

(21) Anmeldenummer: **82105566.2**

(22) Anmeldetag: **24.06.82**

(54) **Verfahren zur Herstellung von 2,4-Dihydroxypyrimidin (Uracil).**

(30) Priorität: **23.07.81 DE 3130455**

(43) Veröffentlichungstag der Anmeldung: **09.02.83 Patentblatt 83/6**

(45) Bekanntmachung des Hinweises auf die Patenterteilung: **08.01.86 Patentblatt 86/2**

(84) Benannte Vertragsstaaten: **AT BE CH DE FR GB IT LI LU NL SE**

(56) Entgegenhaltungen:
**US - A - 3 718 649**

**JOURNAL OF THE AMERICAN CHEMICAL SOCIETY, Band 48, 1926, Easton D. DAVIDSON et al. "The Preparation of Uracil from Urea" Seiten 2379 bis 2383**
**Chemical Abstracts Band 85, Nr. 5, 2. August 1976, Columbus, Ohio, USA I. BASNAK et al. "Nucleic acid components and their analogs. Part CLXXIX. The synthesis of 5-cyclopropyluracil" Seite 394, Spalte 1, abstract Nr. 32945h**
**BEILSTEINS HANDBUCH DER ORGANISCHEN CHEMIE 4. Auflage, Band 24, 1. Ergänzungswerk 1936, VERLAG VON JULIUS SPRINGER Seite 312**

**Die Akte enthält technische Angaben, die nach dem Eingang der Anmeldung eingereicht wurden und die nicht in dieser Patentschrift enthalten sind.**

(73) Patentinhaber: **DYNAMIT NOBEL AKTIENGESELLSCHAFT, Postfach 1209, D-5210 Troisdorf, Bez. Köln (DE)**

(72) Erfinder: **Peeters, Hermann, Dr., Porzer Strasse 1, D-5216 Niederkassel (DE)**

**EP 0 071 018 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Herstellung von 2,4-Dihydroxypyrimidin (Uracil) aus Alkaliformylessigester in einer 4-Stufensynthese ohne Isolierung von Zwischenprodukten nach dem Reaktionsschema:

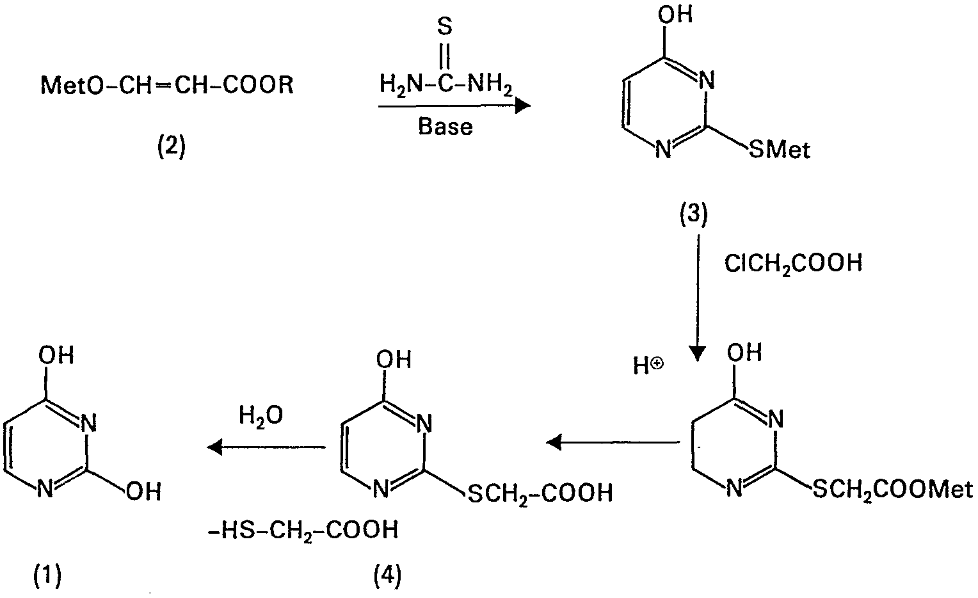


(2) (3) (4) (1)

Uracil ist ein Produkt mit grosser Anwendungsbreite als Industriechemikalie, in der Biomedizin oder als Agrochemikalie [Ind. Chem. Prod. Res. Dev. Vol. 17, No. 4 (1978)]. 2-Carboxymethylthio-4-hydroxy-pyrimidin ist ein chemisches Zwischenprodukt, das in Uracil überführt werden kann oder als Ausgangsprodukt für verschiedene pharmazeutisch wirksame Pyrimidinderivate dienen kann.

Uracil kann auf verschiedene Weise hergestellt werden. Durch Umsetzung von Äpfelsäure mit Harnstoff in Gegenwart von 115% Oleum [J. Amer. Chem. Soc. 48 (1926) 2379] werden geringe Ausbeuten (~55%) erhalten. Grosse Mengen verdünnter Schwefelsäure fallen an und sind nachteilig.

Die Darstellung von Uracil durch Erhitzen von 2-Carboxymethylthio-4-hydroxypyrimidin mit Wasser, ebenso durch Umsetzung von 2-Mercapto-4-hydroxypyrimidin mit Chloressigsäure [Am. Chem. J. 40 (1908) 547] ist bereits beschrieben.

2-Carboxymethylthio-4-hydroxypyrimidin kann durch Umsetzung von 2-Mercapto-4-hydroxypyrimidin in wässrig alkalischem Medium mit Chloressigsäure und anschliessendes Ansäuern in 70% Ausbeute erhalten werden [C.A. 52 (1958) 18702 h].

Die Synthese von Uracil und Carboxymethylthiopyrimidin ist nur ausgehend von reinem 2-Mercapto-4-hydroxy-pyrimidin beschrieben.

Es bestand daher die Aufgabe, ein einfaches Verfahren zur Herstellung von Uracil und von 2-Carboxymethylthiopyrimidin zu finden, das ausgehend von Alkaliformylessigsäurealkylester ohne Isolierung von Zwischenstufen direkt zum Endprodukt führt, was einen erheblichen verfahrenstechnischen Vorteil darstellt.

Es wurde gefunden, dass die gestellte Aufgabe einfach und elegant mit überraschend hoher Ausbeute verfahrensgemäss gelöst werden kann. Überraschend behindern die Nebenprodukte nicht die Reaktionen und erfordern nicht die Isolierung von Zwischenstufen.

Demgemäss wird Alkaliformylessigsäurealkylester mit Thioharnstoff zunächst zum Alkalisalz des 4-Mercapto-4-hydroxypyrimidins, beispielsweise nach der US-PS 3 718 649, und dieses direkt mit Chloressigsäure weiter umgesetzt.

Hierzu kann beispielsweise einer wässrigen Lösung aus Alkalihydroxid und Thioharnstoff ein Alkaliformylessigsäurealkylester als Feststoff oder gelöst in wasserhaltigen polaren Lösungsmitteln, bevorzugt in Wasser bei Temperaturen von etwa 10 bis 25°C zudosiert und im Temperaturbereich von 20 bis 180°C in etwa 1 bis 2 Stunden zur Reaktion gebracht werden. Die Menge an Alkalihydroxid sollte 1 bis 8 mol, vorzugsweise 2 bis 5 Mol, pro Mol Thioharnstoff betragen. Die Konzentration der Reaktionslösung bezüglich Thioharnstoff sollte 0,2 bis 6 Mol/l, vorteilhaft 0,5 bis 4 Mol/l, betragen. Das Molverhältnis von Thioharnstoff zu Alkaliformylessigester sollte etwa 1 zu 1 sein.

Im Alkaliformylessigester kann Me ein Alkalimetall, sehr bevorzugt Natrium oder weniger bevorzugt Kalium, und Alkyl ein bevorzugt gesättigter Alkylrest von 1 bis 8 C-Atomen, sehr bevorzugt Methyl oder Ethyl sein.

Als Reaktionsprodukt fällt das Alkalisalz des 4-Hydroxy-2-mercaptopyrimidins an, das gut wasserlöslich ist. Dieses wird direkt unter Kühlung bei etwa 20 bis 30°C mit Chloressigsäure wie oben bereits beschrieben umgesetzt. Eine weitere Alkalizugabe oder Wasserzugabe ist nicht notwendig. Das Molverhältnis von Chloressigsäure zu eingesetztem Thioharnstoff sollte 2 zu 1 bis 0,5

zu 1, vorteilhaft 1,5 zu 1 bis 0,75 zu 1 sein bzw. 3 bis 1 Mol, vorzugsweise 1,5 bis 1 Mol je Mol 4-Hydroxy-2-mercaptopyrimidin-Alkalisalz.

2-Carboxymethylthio-4-hydroxypyrimidin entsteht als Salz, aus dessen Lösung durch Ansäuern mit Mineralsäuren wie Schwefelsäure oder Salzsäure oder mit Chloressigsäure und Einstellung auf einen pH-Wert von ≦7, gegebenenfalls unter pH 3, die freie Verbindung ausgefällt wird.

2-Carboxymethylthio-4-hydroxypyrimidin wird in Gegenwart von Wasser, beispielsweise als Fällung im Fällwasser oder gegebenenfalls noch feucht, weiter zu Uracil umgesetzt.

Es ist auch möglich, den Stoff nach Ausfällung rein zu isolieren und zu trocknen.

Uracil wird dadurch erhalten, dass die Lösung des Alkalisalzes von 2-Carboxymethylthio-4-hydroxypyrimidin mit Säuren, besonders Mineralsäure wie Schwefelsäure oder Salzsäure oder mit Chloressigsäure auf einen pH-Wert von ≦7, vorzugsweise <3, eingestellt und anschliessend auf 50 bis 200°C, vorteilhaft auf 100°C erhitzt wird. Bevorzugt werden 1 bis 30, sehr bevorzugt 1 bis 10 Äquivalente Säure verwendet.

Die Reaktionsdauer beträgt 1 bis höchstens 5 Stunden. Das Uracil fällt als wasserunlösliches Produkt an, das abfiltriert, mit Wasser gewaschen und getrocknet wird. Durch Extraktion der Mutterlauge kann die gebildete Mercaptoessigsäure erhalten werden.

Beispiel 1

12,0 g (0,3 Mol) Natriumhydroxid werden in 80 ml Wasser vorgelegt, 15,2 g (0,2 Mol) Thioharnstoff und portionsweise 32,5 g (0,2 Mol) Natriumformylessigsäureethylester (Gehalt 85%) werden bei 20°C zudosiert. Es wird 2 Stunden bei 20 bis 25°C gerührt. Anschliessend wird unter Kühlung bei 20°C 18,9 g (0,2 Mol) Chloressigsäure, gelöst in 30 ml Wasser, zudosiert und die Mischung 1 Stunde zum Rückfluss erhitzt. Nach dem Abkühlen wird mit 10prozentiger Schwefelsäure bis zum pH-Wert 2 angesäuert, der ausfallende Feststoff abfiltriert, mit Wasser gewaschen und bei 50°C im Vakuum getrocknet.
Ausbeute: 31,5 g (84,7% d.Th.) 2-Carboxymethylthio-4-hydroxypyrimidin.
Schm.: 165–168°C.

Beispiel 2

30 g (0,75 Mol) Natriumhydroxid werden in 200 ml Wasser vorgelegt, 38 g (0,5 Mol) Thioharnstoff und portionsweise 81,3 g (0,5 Mol) Natriumformylessigsäureethylester (Gehalt 85%) werden bei 20°C zudosiert. Es wird 2 Stunden bei 20 bis 25°C gerührt. Anschliessend wird unter Kühlung bei 20°C 47,3 g (0,5 Mol) Chloressigsäure in 75 ml Wasser zudosiert in die Mischung 1 Stunde zum Rückfluss erhitzt. Nach dem Abkühlen wird mit konzentrierter Salzsäure bis zum pH-Wert 2 angesäuert und 3 Stunden zum Rückfluss erhitzt. Der Feststoff wird abfiltriert, mit Wasser gewaschen und bei 120°C im Vakuum getrocknet.
Ausbeute: 47,7 g (85,2% d.Th.) 2,4-Dihydroxy-pyrimidin (Uracil).
Schm.: 338–340°C.

**Patentansprüche**

1. Verfahren zur Herstellung von 2,4-Dihydroxy-pyrimidin (Uracil)

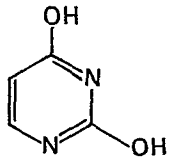

(1),

dadurch gekennzeichnet, dass a) ein Alkaliformylessigsäureester der Formel

MetO–CH=CH–COOR (2),

worin Met ein Alkalimetall, bevorzugt Natrium oder Kalium und R einen Alkylrest, bevorzugt einen Methyl- oder Ethylrest bedeutet, in wässrigem Medium in Anwesenheit von Alkalihydroxid als Base mit Thioharnstoff zum Alkalisalz des 2-Mercapto-4-hydroxypyrimidins der Formel

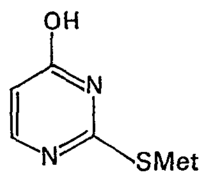

(3),

wobei Met dieselbe Bedeutung wie in der Formel (2) hat, umgesetzt wird, b) dass man die Verbindung (3) anschliessend mit Chloressigsäure zum entsprechenden Alkalisalz von 2-Carboxymethylthio-4-hydroxypyrimidin der Formel

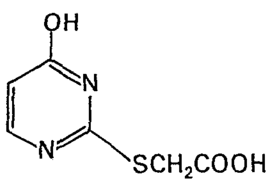

(4),

zur Reaktion bringt, c) dass man durch Ansäuern auf einen pH-Wert von ≦7 die freie Verbindung herstellt, und d) dass man letztere in Uracil durch Erhitzen auf 50 bis 200°C überführt.

2. Verfahren nach Anspruch 1, dadurch gekennzeichnet, dass die Umsetzung zu (3) in einem polaren, wasserhaltigen Lösungsmittel, vorteilhaft in Wasser erfolgt.

3. Verfahren nach den Ansprüchen 1 oder 2, dadurch gekennzeichnet, dass zur Bildung von 2-Carboxymethylthio-4-hydroxypyrimidin 1 bis 8 Äquivalente der Base verwendet werden.

4. Verfahren nach den Ansprüchen 1 oder 3, dadurch gekennzeichnet, dass 1 bis 3, vorzugsweise 1 bis 1,5 Mol Chloressigsäure je Mol 4-Hydroxy-2-mercaptopyrimidin-Alkalisalz verwendet werden.

5. Verfahren nach den Ansprüchen 1 oder 2, dadurch gekennzeichnet, dass die Reaktionstemperatur für die Umsetzung zu (3) 20 bis 180°C beträgt.

6. Verfahren nach mindestens einem der Ansprüche 1 bis 4, dadurch gekennzeichnet, dass die Überführung des Alkalisalzes von 2-Carboxymethylthio-hydroxypyrimidin bei einem pH-Wert von ≦7 erfolgt.

7. Verfahren nach Anspruch 6, dadurch gekennzeichnet, dass die Umsetzung zu Uracil in Gegenwart von Säuren, vorteilhaft von Mineralsäuren, wie Salzsäure oder Schwefelsäure oder von Chloressigsäure erfolgt.

8. Verfahren nach den Ansprüchen 6 oder 7, dadurch gekennzeichnet, dass 1 bis 30, vorzugsweise 1 bis 10 Äquivalente Säure verwendet werden.

9. Verfahren zur Herstellung von 2-Carboxymethylthio-4-hydroxypyrimidin, dadurch gekennzeichnet, dass man die Verfahrensstufen a) bis c) gemäss Anspruch 1 durchführt.

10. Verfahren nach Anspruch 9, dadurch gekennzeichnet, dass 2-Carboxymethylthio-4-hydroxypyrimidin nach Ausfällung isoliert und ggf. getrocknet wird.

**Claims**

1. Process for the preparation of 2,4-dihydroxypyrimidine (uracil)

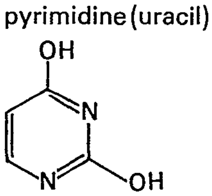

(1),

characterised in that a) an alkali formyl acetic acid ester of the formula

MetO-CH=CH-COOR (2),

wherein Met denotes an alkali metal, preferably sodium or potassium and R an alkyl residue, preferably a methyl or ethyl residue, is reacted in aqueous medium in the presence of alkalihydroxide as base with thiourea to form the alkali salt of 2-mercapto-4-hydroxypyrimidine of the formula

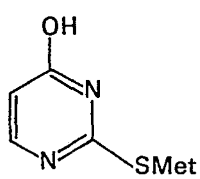

(3),

wherein Met has the same meaning as in formula (2), b) that the compound (3) is brought to reaction next with chloro acetic acid to form the corresponding alkali salt of 2-carboxymethylthio-4-hydroxypyrimidine of the formula

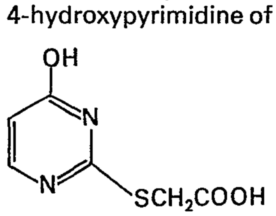

(4),

c) that the free compound is produced by acidification to a pH value of ≦7, and d) the latter is converted to uracil by heating to 50 to 200°C.

2. Process according to claim 1, characterised in that the reaction to (3) takes place in a polar, water-containing solvent, preferably in water.

3. Process according to claims 1 and 2, characterised in that 1 to 8 equivalents of the base are used for formation of 2-carboxymethylthio-4-hydroxypyrimidine.

4. Process according to claims 1 or 3, characterised in that 1 to 3, preferably 1 to 1.5 mol of chloroacetic acid are used per mole of alkali salt of 4-hydroxy-2-mercaptopyrimidine.

5. Process according to claims 1 or 2, characterised in that the reaction, temperature for the conversion to (3) amounts to 20 to 180°C.

6. Process according to at least one of claims 1 to 4, characterised in that the conversion of the alkali salt of 2-carboxymethylthio-hydroxypyrimidine takes place at a pH value of ≦7.

7. Process according to claim 6, characterised in that the reaction to form uracil takes place in the presence of acids, advantageously of mineral acids such as hydrochloric acid or sulphuric acid, or of chloroacetic acid.

8. Process according to claim 6 or 7, characterised in that 1 to 30, preferably 1 to 10 equivalents of acid are used.

9. Process for the preparation of 2-carboxymethylthio-4-hydroxypyrimidine, characterised in that process steps a) to c) are carried out according to claim 1.

10. Process according to claim 9, characterised in that 2-carboxymethylthio-4-hydroxypyrimidine is isolated after its precipitation and is optionally dried.

**Revendications**

1. Procédé pour la préparation de 2,4-dihydroxypyrimidine (uracile)

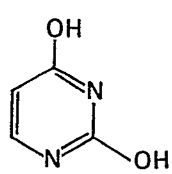

(1),

caractérisé en ce que a) un ester d'acide formylacétique alcalin de formule

MetO-CH=CH-COOR (2)

dans laquelle Met représente un métal alcalin, de préférence le sodium ou le potassium et R un reste alkyle, de préférence un reste méthyle ou éthyle, est transformé avec de la thiourée en milieu aqueux en présence d'un hydroxyde alcalin comme base en le sel alcalin de la 2-mercapto-4-hydroxypyrimidine de formule

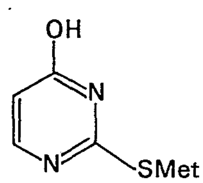

(3),

dans laquelle Met a la même signification que dans la formule (2), b) qu'ensuite le composé (3) est mis à réagir avec de l'acide chloracétique en donnant le sel alcalin correspondant de la 2-carboxyméthylthio-4-hydroxypyrimidine de formule

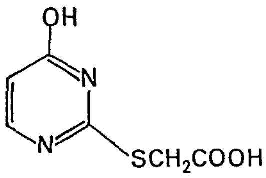

(4),

c) que l'on prépare le composé libre par acidification jusqu'à une valeur de pH ≦7 et d) transforme ce dernier en uracile par chauffage à 50 jusqu'à 200°C.

2. Procédé selon la revendication 1, caractérisé en ce que la transformation en (3) s'effectue dans un solvant polaire aqueux, avantageusement dans l'eau.

3. Procédé selon la revendication 1 ou 2, caractérisé en ce que 1 à 8 équivalents de la base sont utilisés pour la formation de la 2-carboxyméthylthio-4-hydroxypyrimidine.

4. Procédé selon la revendication 1 ou 3, caractérisé en ce que 1 à 3, de préférence 1 à 1,5 moles d'acide chloracétique sont utilisées par mole de sel alcalin de 4-hydroxy-2-mercaptopyrimidine.

5. Procédé selon la revendication 1 ou 2, caractérisé en ce que la température de réaction pour la conversion en (3) s'élève à 20–180°C.

6. Procédé selon l'une quelconque des revendications 1 à 4, caractérisé en ce que la transformation du sel alcalin de la 2-carboxyméthylthiohydroxypyrimidine s'effectue à une valeur de pH ≦7.

7. Procédé selon la revendication 6, caractérisé en ce que la transformation en uracile a lieu en présence d'acides, avantageusement en présence d'acides minéraux tels que l'acide chlorhydrique ou l'acide sulfurique, ou d'acide chloracétique.

8. Procédé selon la revendication 6 ou 7, caractérisé en ce qu'on emploie 1 à 30, de préférence 1 à 10 équivalents d'acide.

9. Procédé pour la préparation de 2-carboxyméthylthio-4-hydroxypyrimidine, caractérisé en ce que l'on conduit les stades opératoires a) à c) conformément à la revendication 1.

10. Procédé selon la revendication 9, caractérisé en ce que la 2-carboxyméthylthio-4-hydroxypyrimidine est isolée après précipitation et est éventuellement séchée.